(19) Europäisches Patentamt
European Patent Office
Office européen des brevets

(11) EP 0 629 397 B1

(12) **FASCICULE DE BREVET EUROPEEN**

(45) Date de publication et mention de la délivrance du brevet: **22.07.1998 Bulletin 1998/30**

(51) Int. Cl.$^6$: **A61K 7/48**

(21) Numéro de dépôt: **94401331.7**

(22) Date de dépôt: **14.06.1994**

# (54) Composition cosmétique ou pharmaceutique anti-radicaux libres pour application topique

Kosmetisches oder pharmazeutisches Präparat zur topischer Verwendung gegen freien Radikale

Cosmetic or pharmaceutical composition for topical application active against free radicals

(84) Etats contractants désignés:
**AT BE CH DE ES FR GB GR IT LI LU NL SE**

(30) Priorité: **15.06.1993 FR 9307205**

(43) Date de publication de la demande:
**21.12.1994 Bulletin 1994/51**

(73) Titulaire:
**LABORATOIRES Dr. N.G. PAYOT**
**75001 Paris (FR)**

(72) Inventeurs:
- **Brin, André-Jean**
  **F-78290 Croissy sur Seine (FR)**
- **Goutelard, Nadine,**
  **F-95270 Chaumontel (FR)**

(74) Mandataire:
**Boulinguiez, Didier et al**
**Cabinet Plasseraud**
**84, rue d'Amsterdam**
**75440 Paris Cedex 09 (FR)**

(56) Documents cités:
**BE-A- 903 646**
**FR-A- 2 490 492**
**FR-A- 2 657 012**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 629 397 B1

## Description

La présente invention a pour objet une composition cosmétique ou pharmaceutique pour application topique, comprenant un extrait hydroglycolique d'algues Chlorella, Scenedesmus et Spiruline en association avec un extrait de café vert.

Elle vise également l'utilisation, pour la préparation de compositions cosmétiques ou pharmaceutiques destinées au traitement de la peau ou du système capillaire, d'un extrait hydroglycolique d'algues chlorella, Scenedesmus et Spiruline en association avec un extrait de café vert.

Enfin, elle concerne un procédé de traitement cosmétique de la peau et du système capillaire.

La peau et les cheveux sont l'objet d'agressions répétées de différents agents oxydants qui, par leur action directe ou indirecte, provoquent des effets indésirables et nocifs, dont notamment un vieillissement cutané accéléré.

Les effets nocifs qu'entraînent les radicaux libres, entités chimiques éphémères au sein de l'organisme, y compris la peau, ont été décrits à maintes reprises dans la littérature médicale, dermatologique et cosmétique. Les radicaux libres peuvent aussi se former en présence d'oxygène et de rayonnement ultra-violet et réagissent notamment avec les acides gras polyinsaturés, éléments fondamentaux de l'architecture cellulaire, provoquant par voie de conséquence de profondes altérations au niveau de l'épiderme. Les glycolipides, sphingolipides et phospholipides, constituants essentiels des membranes, renferment en effet un grand nombre d'acides gras polyinsaturés susceptibles de subir l'action radicalaire. Ainsi, les foyers de peroxydation, en se multipliant, entraînent une instabilité de la couche granulaire et de la barrière cutanée.

De même, la peau et les cheveux peuvent subir des effets néfastes du fait des rayonnements solaires ou de la part de certains agents présents dans l'air atmosphérique comme par exemple le dioxyde de soufre, le dioxyde d'azote, les hydrocarbures, les dérivés halogénés ou le gaz carbonique.

On recherche de ce fait des compositions permettant de protéger efficacement la peau ou le système capillaire contre les susdites agressions et on recherche notamment à l'heure actuelle des compositions pharmaceutiques ou cosmétiques ayant une activité satisfaisante contre les radicaux libres.

Or, la Société Demanderesse a trouvé que, de manière surprenante et inattendue, l'association d'un extrait hydroglycolique de micro-algues Chlorella, Scenedesmus et Spiruline et d'un extrait de café vert s'avère très active et très performante dans la protection de l'épiderme contre les actions radicalaires mais aussi contre la formation des radicaux libres. De façon surprenante et inattendue, cette association s'est également avérée posséder une activité anti-inflammatoire très importante.

Les compositions pharmaceutiques et cosmétiques conformes à l'invention sont donc caractérisées par le fait qu'elle contiennent une quantité efficace d'au moins un extrait hydroglycolique d'algues Chlorella, Scenedesmus et Spiruline et d'au moins un extrait de café vert.

L'extrait hydroglycolique d'algues Chlorella, Scenedesmus et Spiruline est obtenu par un procédé d'extraction ménagée des composants des micro-algues Spiruline, Chlorella et Scenedesmus dans un milieu hydroglycolique.

La Spiruline fait partie de la classe des Cyanophycées. C'est une algue d'eau douce dont la composition qualitative est sensiblement équivalente à celle des algues marines. Il existe cependant des différences marquantes sur le plan quantitatif. Ainsi le taux de glucides est de 18 % au lieu de 60 % pour les algues marines, le taux de lipides est plus élevé : 8 % (au lieu de 5 %), avec 6,5 % d'acides gras essentiels insaturés (dont un fort pourcentage d'acide gamma-linoléique) et 1,5 % d'insaponifiables. Le taux de protides est également très différent puisqu'il est de 65 à 70 %, au lieu de 10 %, ce qui fait que cette algue constitue une source naturelle très riche en tous les acides aminés essentiels, ceux-ci étant présents de plus à des taux élevés. Enfin, la grande richesse vitaminique, et plus particulièrement en vitamine B12 ainsi qu'en provitamine A et en vitamine E, rend cette algue intéressante.

La Chlorella appartient à la classe des Chlorophycées. Il s'agit d'algues vertes répandues dans les lacs et les étangs du monde entier qui renferment une très forte teneur en chlorophylle, environ dix fois supérieure à celle des Spirulines.

Le Scenedesmus appartient à la même classe que la Chlorella. Il s'agit également d'une algue d'eau douce.

Ces deux algues, Chlorella et Scenedesmus, présentent l'avantage d'offrir un apport intéressant en vitamines hydrosolubles B1, B2 et PP. La sélection de ces trois algues Spiruline, Chlorella et Scenedesmus permet d'obtenir un extrait présentant, de façon surprenante et inattendue, une grande activité contre les radicaux libres et présentant d'autres qualités intéressantes pour la cosmétologie.

De préférence, l'extrait d'algues utilisé conformément à l'invention est obtenu par extraction dans un milieu hydroalcoolique comportant un rapport eau/glycol compris entre 5/95 et 95/5 et de préférence entre 10/90 et 90/10. Les glycols préférés sont la glycérine et le propylène glycol.

L'extraction est réalisée avec un rapport solvant hydroalcoolique/algues compris entre 2/1 et 10/1 et de préférence compris entre 3/1 et 8/1.

Afin d'obtenir l'efficacité la plus élevée possible, chacune des trois algues est présente dans le mélange soumis à l'extraction hydroalcoolique avec un pourcentage d'au moins 5 % et de préférence d'au moins 10 %.

Après extraction, une purification est effectuée par précipitation sélective et l'extrait est ensuite filtré sur filtre à 0,22 microns et conservé.

L'extrait de café vert utilisé conformément à l'invention est une fraction purifiée d'un extrait de grain de café vert (Coffea arabica Rubiacées). Les composés principaux sont des acides phénoliques dont la structure chimique de base est la suivante :

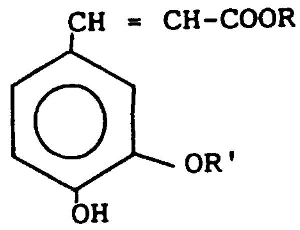

L'extrait de café vert présente une concentration en acide caféique supérieure à 40 %, de préférence supérieure à 45 %, et plus préférentiellement encore supérieure à 50 % sur la matière sèche de l'extrait.

Selon une réalisation préférentielle, l'extrait de café vert utilisé conformément à l'invention contient de 40 % à 85 %, de préférence de 50 à 80 % et plus préférentiellement encore de 60 à 77 % d'acide caféique, et de 1 à 10 %, de préférence de 2 à 8 % d'acide chlorogénique.

L'extrait de café vert en question se présente généralement sous la forme d'une poudre blanchâtre, légèrement aromatique, atomisée sur support maltodextrine. Une analyse type en est la suivante :

| | |
|---|---|
| Acide caféique | 50 - 55 % |
| Acide chlorogénique | 2 - 5 % |
| Autres acides phénoliques | 2 - 5 % |
| Maltodextrine | 15 - 22 % |
| Eau | 5 % max. |

L'absorption molaire de la plupart des molécules présentes dans l'extrait de café vert est supérieure à 20 000 à 321 nm, conférant donc à cet extrait un coefficient d'extinction à 321 nm d'environ 500. Cette valeur relativement élevée fait que l'extrait possède de bonnes propriétés de protection vis-à-vis du rayonnement UV et surtout des UVB.

L'effet de synergie obtenu par l'association de l'extrait d'algues chlorella, Scenedesmus et Spiruline avec l'extrait de café vert est surtout marqué lorsque le rapport pondéral de l'extrait d'algues sur l'extrait de café vert est compris entre 1/1 et 30/1, de préférence compris entre 2/1 et 20/1 et plus préférentiellement encore compris entre 3/1 et 15/1.

L'association binaire anti-radicaux libres conforme à l'invention peut être utilisée dans les compositions cosmétiques ou pharmaceutiques destinées au traitement de la peau ou du système capillaire, ou aux traitements anti-inflammatoires. L'invention vise donc des compositions cosmétiques ou pharmaceutiques, caractérisées en ce qu'elles contiennent une quantité efficace d'au moins un extrait hydroglycolique d'algues chlorella, Scenedesmus et Spiruline et d'au moins un extrait de café vert.

On entend par quantité efficace la quantité permettant d'obtenir l'effet protecteur et/ou anti-inflammatoire recherché. Le plus généralement, cette quantité est comprise entre 0,01 % et 8 % en poids, de préférence entre 0,02 % et 5 % et plus préférentiellement encore entre 0,05 et 5 % en poids pour l'extrait de café vert et entre 0,1 % et 15 %, de préférence entre 0,2 % et 12 % et plus préférentiellement encore entre 0,5 % et 8 % en poids pour l'extrait d'algues Chlorella, Scenedesmus et Spiruline.

Les compositions selon l'invention destinées à une application topique peuvent être notamment des solutions ou dispersions du type lotion ou sérum, des émulsions de consistance liquide ou semi-liquide du type lait, obtenues par dispersion d'une phase grasse dans une phase aqueuse (H/E) ou inversement (E/H), ou des suspensions ou des émulsions de consistance molle du type crème ou gel, ou encore des microsphères, microgranulés ou des dispersions vésiculaires de type ionique et/ou non ionique.

Ces compositions peuvent être préparées de façon connue en soi et constituent notamment des crèmes de nettoyage, de protection ou de soin pour le visage, pour les mains ou pour le corps, par exemple crème de jour, crème de

nuit, crème démaquillante, crème fond de teint, crème anti-solaire, des fonds de teint fluides, des crèmes ou des pommades anti-inflammatoires, des laits de démaquillage, des laits corporels de protection ou de soin, des laits anti-solaires, des lotions de nettoyage, des lotions anti-solaires, des lotions de bronzage artificiel, des compositions pour bain, des compositions désodorisantes contenant un agent bactéricide, des shampoings, des lotions pour les cheveux, des crèmes pour les cheveux. Ces compositions peuvent également consister en une préparation solide, par exemple des savons. Les compositions qui sont de nature fluide peuvent être quant à elle présentées sous forme de bombe aérosol.

Selon une réalisation préférentielle de l'invention, les compositions conformes à l'invention contiennent, outre l'association binaire de l'extrait hydroalcoolique d'algues chlorella, Scenedesmus et Spiruline et d'extrait de café vert, une quantité de 0,01 % à 6 % en poids de tocophérols, et en particulier de linoléate de tocophérol. Le linoléate de tocophérol est un mélange de plusieurs esters d'acides gras de tocophérols. Il contient un minimum de 51 % de linoléate de tocophérol, un minimum de 25 % d'oléate de tocophérol, du palmitate de tocophérol et du myristate de tocophérol.

Il constitue un facteur anti-radicaux libres et apporte à la peau un des acides gras essentiels : l'acide linoléique, mais il agit également en tant que facteur d'hydratation et empêche le durcissement du stratum corneum résultant de l'exposition aux UV. Son utilisation est donc particulièrement préconisée dans les produits pré-solaires et solaires.

Outre l'association binaire conforme à l'invention et outre éventuellement le linoléate de tocophérol, les compositions selon l'invention peuvent contenir tous types d'ingrédients actifs ou d'excipients utilisés de façon conventionnelle dans les compositions cosmétiques ou pharmaceutiques destinées à la peau ou au système capillaire.

Avantageusement, les compositions conformes à l'invention comportent un complexant, tel que l'EDTA disodique, visant à complexer les ions bivalents ou trivalents qui catalysent les réactions d'oxydation.

Des filtres solaires, de préférence non proradicalaires tels que ceux commercialisés sous les marques EUSOLEX 6300 ou UVINUL MS40, peuvent être présents dans lesdites compositions. De même, celles-ci peuvent contenir des pigments tels que l'oxyde de titane, éventuellement enrobé, par exemple au silicone, du micatitane, éventuellement enrobé, du mica également éventuellement enrobé ou du polyméthylméthacrylate.

Les quantités généralement utilisées de ces différents ingrédients sont de 0,01 à 5 % en poids pour les filtres solaires, de 0,1 % à 5 % en poids pour les pigments et de 0,001 % à 0,1 % pour un complexant tel que l'EDTA sodique.

Comme dans tous les produits cosmétiques, des émollients, des parfums, des conservateurs, des colorants, et des agents émulsifiants peuvent en outre être utilisés si nécessaire.

Les compositions cosmétiques selon l'invention peuvent être aussi bien des compositions prêtes à l'emploi que des solutions ou des pâtes concentrées devant être diluées avant utilisation.

L'invention vise également l'utilisation, pour la préparation de compositions cosmétiques ou pharmaceutiques destinées au traitement de la peau ou du système capillaire, ou aux traitements anti-inflammatoires, de l'association d'un extrait hydroglycolique d'algues Chlorella, Scenedesmus et Spiruline et d'un extrait de café vert.

Elle a en outre pour objet un traitement cosmétique caractérisé par le fait que l'on applique sur la peau ou sur le système capillaire une composition contenant au moins un extrait hydroglycolique d'algues Chlorella, Scenedesmus et Spiruline et au moins un extrait de café vert.

Le procédé de traitement cosmétique conforme à l'invention peut être mis en oeuvre par application des compositions cosmétiques telles que définies précédemment, selon les techniques d'utilisation conventionnelles de ces compositions. Ce procédé de traitement cosmétique est mis en oeuvre de façon à appliquer une quantité efficace de l'association conforme à l'invention, c'est-à-dire en fait une quantité suffisante pour obtenir l'effet de protection recherché.

Ce procédé de traitement cosmétique est destiné à protéger la peau ou le système capillaire contre les éléments d'agressions extérieurs tels que les agents oxydants, les rayonnements solaires, les agents polluants et est destiné à maintenir la structure de la peau ou des cheveux et à améliorer les qualités de la peau.

L'invention pourra être mieux comprise à l'aide des tests d'évaluation et des exemples d'application qui suivent, donnés à titre illustratif.

EXEMPLE 1 : EVALUATION DU POTENTIEL ANTI-RADICALAIRE DE L'EXTRAIT D'ALGUES UTILISE SELON L'INVENTION

1. Protection "in vitro" contre la peroxydation lipidique

Une dispersion de phospholipides à 3 % sous forme de liposomes est soumise à une agression radicalaire selon un protocole donné (voir ci-dessous). Dans ces conditions, on évalue le pouvoir anti-radicalaire d'un produit par sa capacité à inhiber la dégradation lipidique. Cette dégradation lipidique peut être mesurée par spectrophotométrie UV avec l'apparition d'un maximum d'absorption à 233 nm, correspondant aux diènes conjugués.

Le protocole est le suivant :

On ajoute, à une solution liposomale à 3 %, 0,02 % de $FeCl_2$ et 0,3 % de KSCN ainsi que de l'eau oxygénée à 110 volumes diluée 10 fois.

Un échantillon avant addition de $FeCl_2$ est gardé au froid en l'absence d'agent oxydant, pour servir de référence au spectrophotomètre. Les échantillons à tester sont laissés six heures au bain-marie à 45°C et une nuit à température ambiante. La manipulation a été faite sans conservateur.

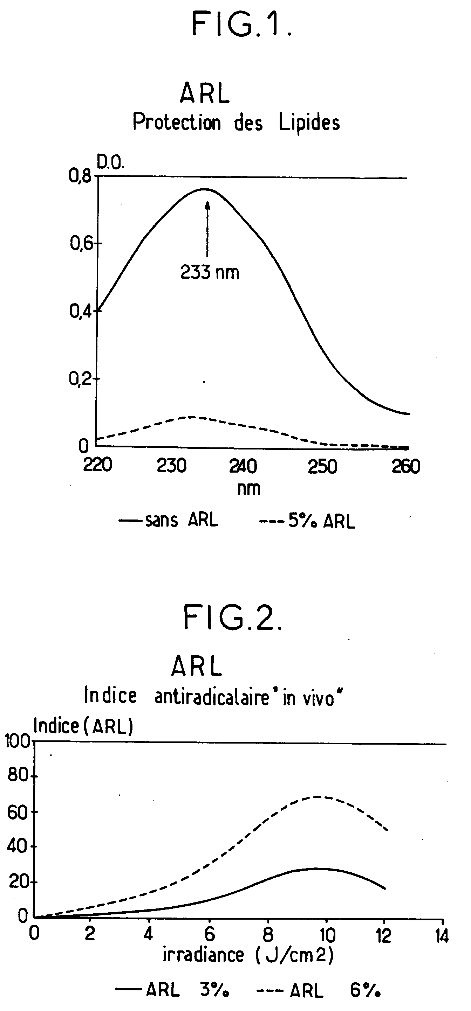

Les courbes présentées sur la figure 1 montre que l'échantillon témoin (ne contenant pas d'extrait d'algues) est fortement oxydé puisqu'on relève un pic caractéristique à 233 nm des diènes conjugués. La courbe du mélange testé, contenant 5 % d'extrait hydroglycolique d'algues Spiruline, Chlorella, Scenedesmus (ci-après dénommé extrait ARL) montre la très bonne inhibition de la lipoperoxydation, celle-ci étant supérieure à 85 %.

2. Protection anti-radicaux libres "in vivo"

L'évaluation du pouvoir anti-radicaux libres "in vivo" sur la peau, au niveau de l'avant-bras est réalisée en suivant la décoloration du β-carotène par un rayonnement UVA en fonction du temps, et en présence ou en l'absence d'extrait ARL. L'action des radicaux libres entraîne une décoloration du β-carotène par oxydation.

Après préparation de la peau (avant-bras) par un nettoyage à l'éther, on applique par un léger massage une quantité mesurée (50 μl) du produit (sous forme de crème ou de lait) contenant le principe actif à tester sur une surface de 5 $cm^2$.

On mesure la couleur de la peau au niveau des endroits traités, à l'aide d'un chromamètre de marque MINOLTA (valeur $B^0$).

Trente minutes plus tard, on applique une solution contenant du β-carotène sur chaque site et on mesure de nouveau la couleur de la peau (valeur $B^{100}$).

L'avant-bras est alors exposé à l'irradiation UVA (chaque irradiation correspondant à une quantité de 2 joules/$cm^2$). Après chaque traitement, on mesure la couleur de la peau (valeur $B^2$, $B^4$, $B^6$, $B^8$, $B^{10}$ et $B^{12}$). A partir de ces valeurs lues au chromamètre, on calcule l'indice de couleur selon la formule :

$$I = 100 - [\frac{B^{100} - B^n}{B^{100} - B^0} \times 100]$$

avec n = 2, 4, 6, 8, 10, 12.

Dans tous les cas de figure, cet indice de couleur diminue avec la quantité d'irradiance croissante. Pour mieux apprécier l'effet protecteur de l'ARL, on calcule l'indice de protection anti-radicaux libres selon la formule :

$$I^{ARL} = \frac{I_c^{\,x} - I_c^{\,p}}{I_c^{\,p}} \times 100$$

où x est le produit à tester et p un placebo.

L'étude a été effectuée sur cinq volontaires âgés de 27 à 42 ans (2 hommes, 3 femmes). L'utilisation des deux bras permet de doubler le nombre de mesures. On compare l'efficacité de deux crèmes H/E contenant respectivement 3 et 6 % de l'ARL avec celui d'une crème placebo.

La figure 2 montre les indices de protection anti-radicaux libres $I^{ARL}$ des deux concentrations de l'ARL.

On constate que l'efficacité augmente avec la dose d'irradiation.

Ces essais montrent que l'ARL possède des qualités anti-radicalaires et protectrices, en piégeant les radicaux libres dès leur formation et en bloquant les réactions radicalaires en cascade.

EXEMPLE 2 : COMPARAISON DU POTENTIEL ANTI-RADICALAIRE DE L'ARL ET D'UNE SUPEROXYDE-DISMUTASE (SOD)

L'activité anti-radicalaire de l'ARL a été évaluée en comparaison avec un produit connu pour ses bonnes propriétés anti-radicalaires, en l'occurrence la superoxyde dismutase, dénommée ci-après SOD (produit commercialisé par exemple par la Société SIGMA sous la référence S 3589).

Le principe de la méthode repose sur l'évaluation comparée de la cytotoxicité induite par le système hypoxanthine xanthine oxydase (HX-XO) en présence de différentes concentrations du principe actif étudié. Le système enzymatique HX-XO génère la plupart des radicaux oxygénés. Cependant, différents travaux ont montré que $H_2O_2$ est le principal agresseur.

Le protocole expérimental est le suivant. Des fibroblastes murins (lignée L929) sont ensemencés dans des microplaques à 96 puits à raison de 25 000 cellules/puits. 24 heures après ensemencement, le milieu de culture est éliminé

et remplacé par du milieu neuf. Les plaques sont replacées à l'étude à 37°C pendant 48 heures.

Après cette période d'incubation, le milieu de culture est éliminé. Les cellules sont exposées aux radicaux libres par l'intermédiaire du système HX-XO : 100 µl d'une solution de XO à 8 mU/ml préparée dans du tampon PBS+ sont ajoutés dans chaque puits ainsi que 100 µl d'une solution de HX à concentrations croissantes.

Après 2 heures 30 d'incubation, les plaques sont vidées, rincées et reçoivent de nouveau du milieu de culture. Elles sont ensuite replacées à l'étude pendant 24 heures. Après incubation, la viabilité cellulaire est évaluée par un test au MTT 3(4,5-dimethylthiazol-2-yl)-2,5 diphenyl tetrazolium bromide. Le principe de ce test repose sur le fait que le MTT est réduit par la succinyl déshydrogénase mitochondriale des cellules métaboliquement actives, en formazan bleu violet insoluble. Le principe du test consiste donc, après addition du MTT et incubation de 3 heures, en la mise en solution du formazan produit. La densité optique de la solution bleu-violet est ensuite mesurée par spectrophotométrie. Celle-ci est donc proportionnelle au nombre de cellules métaboliquement actives.

Une concentration d'hypoxanthine de 15 µg/l a été retenue pour l'étude de la recherche de l'effet protecteur.

<u>Effet protecteur des principes actifs hydrosolubles étudiés : ARL et SOD</u>

L'effet protecteur a été évalué selon le protocole expérimental suivant. Des fibroblastes murins (lignée L929) sont ensemencés dans des microplaques à 96 puits, à raison de 25 000 cellules/puits. 24 heures après ensemencement, le milieu est éliminé et remplacé par du milieu contenant le produit étudié (traitement "préventif").

Trois doses, pour chaque principe actif, ont été appliquées : DNC, 1/2 DNC et 1/10 DNC.

Après 48 heures de "traitement", les plaques sont vidées et soumises à l'action du système HX-XO pendant 2 heures 30 (HX = 15 µg/ml et XO = 8 mU/ml) en présence du principe actif étudié (traitement "immédiat").

Après les 2 heures 30 d'incubation, les plaques sont vidées et rincées afin d'éliminer le système générateur de radicaux libres. Les cellules reçoivent ensuite du milieu neuf renfermant le produit (traitement "curatif").

Après 24 heures d'incubation, la viabilité cellulaire des cultures "témoins" et des cellules "traitées" est évaluée par un test de viabilité cellulaire au MTT.

Le pourcentage de toxicité est calculé selon les formules :

$$A = \frac{\text{DO puits non traités - (DO puits non traités + HX)}}{\text{DO puits non traités}} \times 100$$

et

$$B = \frac{\text{DO puits traités - (DO puits traités + HX)}}{\text{DO puits traités}} \times 100$$

lorsque les cellules sont "traitées" par une substance donnée.

Le pourcentage d'activité protectrice du produit étudié est ensuite déterminé par la formule :

$$\text{Pourcentage d'activité} = \frac{A - B}{A} \times 100$$

Le tableau 1 ci-après présente les résultats obtenus pour l'ARL d'une part et la SOD d'autre part.

TABLEAU 1

| | ARL | | | SOD | | |
|---|---|---|---|---|---|---|
| CONCENTRATION | 1/10 | 1/2 | DNC | 1/10 | 1/2 | DNC |
| % ACTIVE | 31 | 41 | 71 | 29 | 75 | 91 |
| TEST | NS | S | S | NS | S | S |
| (S : statistiquement significatif ($p < 0.05$)<br>Test : analyse de variance et test de Dunnett.) | | | | | | |

On peut constater que les deux produits étudiés présentent une bonne activité anti-radicaux libres. Le produit ARL présente cependant une activité inférieure à celle de la SOD. Pour les deux produits, l'activité protectrice est dépendante de la dose administrée.

EXEMPLE 3 : EVALUATION DU POTENTIEL ANTI-RADICALAIRE DE L'EXTRAIT DE CAFE VERT

Cette évaluation a été effectuée en comparant la cytotoxicité induite par les rayonnements ultraviolets (UVB) sur des cultures témoins et sur des cultures traitées par différentes concentrations de l'extrait de café vert (ci-après dénommé ECV).

Cytotoxicité des irradiations UV

Le protocole expérimental est le suivant.

Des fibroblastes murins (lignée L929) sont ensemencés à raison de 30 000 cellules/puits dans des microplaques de culture à 96 puits. 24 heures après ensemencement, le milieu de culture est éliminé et remplacé par du milieu neuf. Les plaques sont remises à incuber pendant 48 heures en étuve à CO2. Après cette incubation, les plaques sont vidées. Les cellules sont soumises à une irradiation UVB (tube Philips TL 20 W/12) à travers une solution saline de Hank's sans Rouge de Phénol.

Après l'irradiation, les plaques sont vidées et reçoivent du milieu de culture neuf. Après une incubation de 24 heures en étude à CO2, la viabilité cellulaire est évaluée par un test colorimétrique au MTT.

Il existe une relation linéaire entre la mortalité cellulaire et le temps d'irradiation, exprimé en log :

$$\text{Prob (\% mort.)} = 1.309 \log (\text{temps}) + 4.167.$$

Une droite de régression permet de calculer le temps d'exposition entraînant 50 % de mortalité cellulaire (TL50 = 4 min) ou le temps d'exposition entraînant 70 % de mortalité (TL70 = 11 min).

Activité protectrice de l'ECV

Le protocole expérimental est le suivant.

Des fibroblastes murins (lignée L929) sont ensemencés à raison de 30 000 cellules/puits dans des microplaques à 96 puits. 24 heures après ensemencement, le milieu est éliminé et remplacé par du milieu neuf contenant le produit étudié (traitement "préventif"). Trois concentrations de l'ECV sont étudiées : DNC, 1/2 DNC et 1/10 DNC (deux colonnes de 8 puits par concentration).

Après 48 heures de traitement "préventif", les plaques sont vidées et les cellules sont soumises à une irradiation ultraviolette UVB à travers une solution de Hank's sans rouge de phénol et en présence de l'ECV (traitement "immédiat").

Après l'irradiation, la solution saline est éliminée et remplacée par du milieu de culture neuf contenant le produit actif aux différentes concentrations (traitement "curatif") pendant 24 heures.

La viabilité cellulaire est évaluée, en fin d'essai, par un test au MTT.

Le pourcentage de toxicité induite par les UVB est calculé pour chaque concentration de principe actif par rapport à un témoin interne non irradié selon la formule :

$$\text{\% cytotox.} = \frac{\text{DO puits non irradiés - DO puits irradiés}}{\text{DO puits non irradiés}} \times 100$$

Les cultures des lots témoins et traités ont été soumises à deux temps d'exposition aux UVB :

- t1 : 2 min (soit 93 $mJ/cm^2$), temps devant entraîner environ 30 % de mortalité cellulaire dans le lot témoin (d'après l'équation de la droite de régression obtenue précédemment).
- t2 : 2 min (soit 280 $mJ/cm^2$), temps devant entraîner 65 % de mortalité cellulaire sur des cultures non traitées.

Les résultats obtenus sont repris dans le tableau 2 ci-après.

Tableau 2

| | E.C.V. | | |
|---|---|---|---|
| Concentrations | DNC | 1/2 DNC | 1/10 DNC |
| AP1 (93 $mJ/cm^2$) | 100 % | 100 % | 40 % |
| AP2 (280 $mJ/cm^2$) | 100 % | 100 % | 14 % |

On peut constater que l'effet protecteur obtenu à l'aide de l'ECV est total, après une irradiation de 93 $mJ/cm^2$, dans les lots DNC et 1/2 DNC : aucune mortalité cellulaire n'est observée dans ces lots alors qu'on enregistre environ 40 % de mortalité dans le lot témoin non traité. L'effet protecteur reste important à 1/10 DNC.

Des résultats identiques sont obtenus après une irradiation de 280 $mJ/cm^2$ provoquant 67 % de mortalité dans le lot témoin non traité.

On peut en conclure que l'extrait de café vert est doué d'une activité anti-radicalaire importante.

EXEMPLE 4 : EVALUATION DE L'ACTIVITE CAPTEUR DE RADICAUX LIBRES DE PLUSIEURS EXTRAITS VEGETAUX

Pour cette évaluation, la phéomélanine naturelle photoirradiée est utilisée comme modèle générateur de radicaux libres. Elle possède dans sa structure un radical libre intrinsèque et sa photooxydation induit une augmentation du nombre des espèces radicalaires.

La détection est effectuée par une méthode directe la résonance paramagnétique électronique (R.P.E.), méthode spectroscopique de choix pour mettre en évidence les radicaux libres.

L'activité capteur de radicaux libres du produit test est évaluée en déterminant sa capacité à inhiber la production radicalaire.

La phéomélanine naturelle extraite de cheveux roux est utilisée. Il s'agit d'un hétéropolymère, dont le monomère le plus fréquent est une unité 4-hydroxybenzothiazole associée à une protéine.

Plusieurs extraits végétaux, référencés n° 1 à n° 10, ont été testés pour leur éventuelle activité anti-radicalaire. La concentration a été choisie en fonction de l'hydrosolubilité de chaque produit, avec un maximum de 5 %. En ce qui concerne les produits liposolubles (produits référencés 6 et 10), la solubilité a été obtenue à l'aide d'un tensio-actif, le Montanox[R] 80.

Le produit 1 étant très oxydant à 2 %, une concentration de 0,5 % a été retenue.

Un spectrophotomètre PERKIN ELMER est utilisé pour les dosages. Les photolyses sont réalisées par un stimulateur solaire. Il s'agit d'une lampe à arc Xénon.

Un spectromètre ER 200 BRUKER est utilisé pour la détection des radicaux libres stables.

200 µl d'une solution de phéomélanine (2 mg/ml, pH : 7,4) sont introduits dans une cellule en quartz placée dans la cavité R.P.E. Le faisceau lumineux de la lampe est focalisé sur la cavité : l'irradiation se réalise in situ dans l'appareil R.P.E.

Les spectres R.P.E. sont obtenus avant (signal intrinsèque) et après irradiation (signal illuminé).

Une comparaison de l'amplitude du signal illuminé de la phéomélanine, en présence et en absence du produit, est alors effectuée. L'amplitude du signal de la solution de phéomélanine est mesurée avant et après irradiation, d'abord en l'absence du produit testé, puis en présence du produit.

L'amplitude moyenne et l'écart-type des signaux intrinsèques ($A_{IN}$) et illuminés ($A_{ILL}$) de la phéomélanine en présence et en l'absence de produit, sont calculés. L'effet du produit testé est évalué par le calcul suivant et exprimé en pourcentage de variation d'amplitude V :

$$V = \frac{(A_{ILL} - A_{IN})\ \text{produit} - (A_{ILL} - A_{IN})}{(A_{ILL} - A_{IN})} \times 100$$

Les pourcentages de variation de l'amplitude du signal illuminé, en présence des produits testés sont représentés dans le tableau 3 ci-dessous.

TABLEAU 3

| Produits et concentration | | % variation de l'amplitude du signal moyenne σ |
|---|---|---|
| **1** Noix d'Alep Supextract éclaircie | 0,5 % | + 71,35 % +/- 11,71 |
| **2.** Extrait de café vert ERL | 2 % | - 53,43 % +/- 11,36 |
| **3.** Extrait sec de thé vert | 2 % | + 30,77 % +/- 7,42 |
| **4.** Sylmarina fitosoma | 2 % | - 13,13 % +/- 16,18 |
| **5.** Extrait de thé vert | 5 % | + 9,56 % +/- 9,17 |
| **6.** Linoléate de tocophérol | 2 % | - 30,81 % +/- 7,87 |
| **7.** Concentrat de feuilles de mûrier | 5 % | + 8,33 +/- 7,64 |
| **8.** Extrait de Spiruline | 5 % | + 12,03 +/- 9,31 |
| **9.** ARL | 5 % | - 30,20 +/- 10,92 |
| **10.** Epaline | 5 % | - 0,47 % +/- 7,52 |

Une diminution de l'amplitude du signal de la phéomélanine photoirradiée est observée en présence de 5 produits, mais on peut constater que cette diminution n'est réellement significative qu'en présence de 3 produits l'extrait de café vert ERL, le linoléate de tocophérol et l'extrait d'algues ARL.

Ces produits montrent donc un bon effet inhibiteur de la production radicalaire.

Les autres produits entraînent une augmentation du signal de la phéomélanine photoirradiée, en particulier le produit 1 à 0,5 % et le produit 3 à 2 %, ces produits étant pro-oxydants.

EXEMPLE 5 : ACTIVITE RADICALAIRE EN FONCTION DE LA CONCENTRATION ET SYNERGIE ENTRE L'ARL ET L'ECV

La même méthode que précédemment est utilisée et les pourcentages de variation de l'amplitude du signal illuminé en fonction des concentrations des produits sont présentés dans le tableau 4 ci-dessous pour le produit ECV, pour le linoléate de tocophérol et enfin pour l'ARL.

TABLEAU 4

| | 0,50 % | 1 % | 2 % | 4 % | 5 % |
|---|---|---|---|---|---|
| ECV | - 56,36 +/- 4,19 | - 56,17 +/- 6,41 | - 50,19 +/-13,04 | - 61,48 +/-12,51 | -- |
| Linoléate de tocophérol | + 1,73 +/-13,44 | - 6,91 +/-12,16 | - 30,81 +/- 8,80 | -- | -- |
| ARL | - 9,27 +/-10,14 | - 6,37 +/- 2,59 | - 17,9 +/-10,84 | - 29,84 +/- 5,79 | - 30,24 +/- 12,21 |

On peut constater que le produit ECV induit sensiblement la même diminution de la production radicalaire lorsque sa concentration varie entre 0,5 et 4 %.

Le linoléate de tocophérol induit une diminution dose-dépendante de la production radicalaire. Cette diminution n'est significative qu'à partir de 2 %.

Le produit ARL induit une diminution dose-dépendante de la production radicalaire, qui est significative à partir de la concentration de 2 % et qui n'augmente plus à partir de 4 %.

Deux mélanges de ces produits ont ensuite été testés :

- ECV à 0,5 % + linoléate de tocophérol à 2 %
- ECV à 0,5 % + ARL à 4 %.

Les pourcentages de variation de l'amplitude du signal illuminé en fonction de ces associations sont présentés

dans le tableau 5.

TABLEAU 5

| Pourcentage de variation de l'amplitude du signal illuminé en présence des associations de produits | |
|---|---|
| ASSOCIATION | % VARIATION DE L'AMPLITUDE DU SIGNAL : MOYENNE, σ |
| ECV à 0,5 % + linoléate de tocophérol à 2 % | - 23,45 +/- 4,95 |
| ECV à 0,5 % + ARL à 4 % | - 56,33 +/- 4,37 |

On peut constater que les deux associations de produits testés induisent une diminution de la production radicalaire, mais on peut constater également que c'est l'association formée par les produits ECV et ARL qui induit, de loin, la plus forte diminution puisque le pourcentage de variation de l'amplitude du signal illuminé de la phéomélanine en présence de cette association est de - 56,33 % +/-4,37.

L'association formée par l'ECV à 0,5 % et l'ARL à 4 % révèle donc une forte capacité à inhiber la formation de radicaux libres générés par la phéomélanine photoirradiée.

L'intérêt de cette association de composés actifs est la synergisation d'activité entre ces produits, l'ARL agissant par ses propriétés anti-radicalaires et antipollution et protégeant ainsi les cellules de la peau à l'extérieur et à l'intérieur de l'action d'agents oxydants puissants tels que les radicaux hydroxyles et l'extrait de café vert protégeant des attaques radicalaires liées au rayonnement UV.

EXEMPLE 6 : CREME DE JOUR ET CREME DE NUIT

On donne ci-après, à titre d'illustration et sans aucun caractère limitatif, plusieurs exemples de compositions cosmétiques conformes à l'invention.

La première est une crème de jour et la seconde est une crème de nuit, dont les formulations sont indiquées dans le tableau 6 ci-après :

TABLEAU 6

| | Crème de jour | 0,6Crème de nuit |
|---|---|---|
| eau | 63.500 | qsp 100.00 |
| caprylique caprique triglycéride | 4.000 | 7.00 |
| propylène glycol | 2.00 | 2.00 |
| polyéthylène glycol PEG 8 | 1.00 | 1.00 |
| huile minérale | 8.00 | 12.00 |
| polyglycéryl méthacrylate | 10.00 | 10.00 |
| glycéryl stéarate et PEG 100 stéarate | 2.00 | 4.00 |
| diméthicone | 3.00 | 3.00 |
| phénoxyéthanol | 0.400 | 0.40 |
| méthylparaben | 0.300 | 0.300 |
| butylparaben | 0.100 | 0.100 |
| isobutylparaben | 0.050 | 0.05 |
| propylparaben | 0.100 | 0.1 |
| ethylparaben | 0.050 | 0.05 |

TABLEAU 6 (suite)

| | Crème de jour | 0,6Crème de nuit |
|---|---|---|
| linoléate de tocophérol | 0.5 % | 0.5 % |
| extrait ARL | 5 % | 5 % |
| ECV | 1 % | 1 % |

EXEMPLE 7 : FOND DE TEINT

| | |
|---|---|
| - eau qsp | 100.000 |
| - propylène glycol | 10.000 |
| - gomme xanthane | 0.300 |
| - diméthicone | 2.000 |
| - huile minérale | 5.000 |
| - stéarate de sorbitan | 2.00 |
| - stéarate de sorbitan POE | 2.00 |
| - néopentanoate d'isostéaryle | 5.00 |
| - caprylique caprique triglycéride | 4.00 |
| - talc | 2.00 |
| - pigments oxyde de fer (rouge, jaune brun, noir) | 2.50 |
| - oxyde de titane | 8.00 |
| - mica | 1.00 |
| - phénoxyéthanol | 0.400 |
| - méthylparaben | 0.300 |
| - butylparaben | 0.100 |
| - isobutylparaben | 0.050 |
| - propylparaben | 0.100 |
| - éthylparaben | 0.050 |
| - linoléate de tocophérol | 3 % |
| - extrait ARL | 4 % |
| - ECV | 2 % |

EXEMPLE 8

Non seulement l'association de l'ARL et d'extrait de café vert conforme à l'invention présente des propriétés anti-radicalaire et anti-pollution très intéressantes, mais elle présente un effet anti-inflammatoire comme le démontre l'exemple ci-après, réalisé à l'aide de la crème de jour dont la formulation est indiquée dans le tableau 6.

L'effet anti-inflammatoire a été évalué, chez l'homme, à partir d'un modèle d'inflammation au nicotinate de méthyle, par mesure du flux sanguin cutané.

Les produits qui ont été testés sont :

- la crème de jour dont la formulation est indiquée dans le tableau 6, cette crème de jour incluant donc l'association conforme à l'invention,
- une préparation à base d'acide niflumique (Nifluril$^{R}$), qui constitue le témoin positif,
- une peau nue non traitée, qui constitue le témoin négatif.

Les essais ont été réalisés sur 10 sujets volontaires sains préalablement informés de la nature de l'expérimentation et ayant donné leur consentement éclairé (9 femmes et 1 homme, d'âge moyen : 26 ans).

Les variations de la microcirculation cutanée ont été mesurées par vélocimétrie laser doppler (Periflux PF2B).

Protocole d'expérimentation

La zone d'exploration est constituée par la face interne de l'avant bras. Les mesures sont effectuées en atmosphère contrôlée de température égale à 22°C et d'humidité relative de 55 %.

Les sujets sont laissés au repos pendant quinze minutes avant l'enregistrement. Après randomisation, la crème et le témoin positif sont appliqués à raison de 4 $mg/cm^2$. Les essais préliminaires ont permis de déterminer le temps d'occlusion nécessaire pour obtenir une réponse pharmacologique optimale. Ce temps est de 3 heures.

Chaque zone, y compris une zone témoin peau nue, est recouverte d'un pansement occlusif durant 3 heures.

Le pansement occlusif est ensuite enlevé et les zones lavées et essuyées.

Après 5 minutes, 2 $mg/cm^2$ d'une solution aqueuse à 0,5 % de nicotinate de méthyle sont appliqués sur les zones testées afin d'induire l'inflammation.

L'enregistrement du flux sanguin cutané est alors réalisé pendant 30 minutes.

Résultats

Les résultats de l'évaluation in vivo de l'activité sur le flux sanguin cutané des formules sont exprimés par le calcul des aires sous courbes (ASC) par rapport à la ligne de base physiologique mesurée sur une zone non traitée ni occluse.

Les résultats sont présentés sur le tableau 7 ci-dessous.

TABLEAU 7

| Résultats des données d'aires sous courbes brutes | | | | |
|---|---|---|---|---|
| Sujet | Peau nue | Nifluril$^R$ | Crème de jour selon l'invention | Ligne de base |
| 1 | 1229 | 50 | 191.3 | 35 |
| 2 | 502.2 | 74.5 | 132.5 | 54 |
| 3 | 674 | 593 | 529.3 | 27 |
| 4 | 285.8 | 53.6 | 81.2 | 16.2 |
| 5 | 463.5 | 42.9 | 55.6 | 27 |
| 6 | 423 | 113.1 | 108.3 | 16.2 |
| 7 | 675 | 63.3 | 110.4 | 24.3 |
| 8 | 395.7 | 85.4 | 225.5 | 26.5 |
| 9 | 11.7 | 86.8 | 29.8 | 24.3 |
| 10 | 654.3 | 75.7 | 52.8 | 22.7 |
| Moyenne | 539.26 | 123.83 | 151.64 | 27.32 |

Le pourcentage d'inhibition de l'inflammation a été calculé pour la crème de jour comprenant l'association de principes actifs conformes à l'invention et la crème Nifluril$^R$, à l'aide de la formule suivante :

$$\text{\% d'inhibition} = \frac{\text{ASC Peau nue - ASC Produit}}{\text{ASC Peau nue}}$$

Le pourcentage d'inhibition trouvé pour le produit Nifluril$^R$ est de 81.26 %.

Le pourcentage d'inhibition trouvé pour la crème conforme à l'invention est de 75.84 %.

La crème de soin conforme à l'invention diminue donc fortement l'inflammation induite par le nicotinate de méthyle.

Cette inhibition est presque équivalente à celle apportée par la crème anti-inflammatoire Nifluril$^R$, ce qui représente

un résultat tout à fait remarquable.

L'association de principes actifs conformes à l'invention présente donc, outre ses propriétés anti-radicalaires et anti-pollution, une activité très significative et très intéressante sur l'inflammation induite par le nicotinate de méthyle, en réalisant une importante inhibition de la réaction inflammatoire.

## Revendications

1. Composition cosmétique ou pharmaceutique pour application topique, caractérisée par le fait qu'elle contient une quantité efficace d'au moins un extrait hydroglycolique d'algues Chlorella, Scenedesmus et Spiruline et d'au moins un extrait de café vert.

2. Composition cosmétique ou pharmaceutique selon la revendication 1, caractérisée par le fait que le rapport pondéral de l'extrait d'algues sur l'extrait de café vert est compris entre 1/1 et 30/1, de préférence entre 2/1 et 20/1 et plus préférentiellement encore entre 3/1 et 15/1.

3. Composition selon l'une ou l'autre des revendications 1 et 2, caractérisée par le fait qu'elle comprend de 0,01 % à 8 %, de préférence de 0,02 à 5 % et plus préférentiellement encore de 0,05 à 5 % en poids d'extrait de café vert.

4. Composition selon l'une quelconque des revendications 1 à 3, caractérisée par le fait qu'elle contient de 0,1 à 15 %, de préférence de 0,2 à 12 % et plus préférentiellement encore de 0,5 à 8 % en poids d'extrait d'algues.

5. Composition selon l'une quelconque des revendications 1 à 4, caractérisé par le fait que l'extrait d'algues est obtenu par extraction hydroglycolique à partir d'un mélange comportant au moins 5 %, et de préférence au moins 10 %, de chacune des algues Chlorella, Scenedesmus et Spiruline.

6. Composition selon l'une quelconque des revendications 1 à 5, caractérisée par le fait que l'extrait de café vert présente une concentration en acide caféique supérieure à 40 %, de préférence supérieure à 45 %, et plus préférentiellement encore supérieure à 50 % sur la matière sèche de l'extrait.

7. Composition selon l'une quelconque des revendications 1 à 6, caractérisée par le fait qu'elle contient en outre de 0,01 % à 6 % en poids de tocophérols, de préférence constitués par du linoléate de tocophérol.

8. Composition selon l'une quelconque des revendications 1 à 7, caractérisée par le fait qu'elle contient en outre de 0,001 % à 0,1 % d'un complexant, de préférence constitué par l'EDTA sodique.

9. Composition selon l'une quelconque des revendications 1 à 8, caractérisée par le fait qu'elle se présente sous la forme d'une lotion, d'une émulsion, d'un lait, de microsphères, de microgranules, d'une crème, d'un gel, d'un baume ou d'une bombe aérosol, de liposomes, ou de vésicules de type ionique ou non ionique.

10. Utilisation pour la préparation de compositions cosmétiques ou pharmaceutiques destinées au traitement de la peau ou du système capillaire et aux traitements anti-inflammatoires, d'un extrait hydroglycolique d'algues Chlorella, Scenedesmus et Spiruline en association avec un extrait de café vert.

11. Procédé de traitement cosmétique de la peau et du système capillaire, caractérisé par le fait qu'on applique sur la peau ou sur le système capillaire une quantité efficace d'un extrait hydroglycolique d'algues Chlorella, Scenedesmus et Spiruline en association avec un extrait de café vert.

## Claims

1. A cosmetic or pharmaceutical composition for topic application, characterised in that it contains an effective quantity of at least one hydroglycolic extract of chlorella, scenedesmus and spiruline algae and at least one extract of unroasted coffee.

2. A cosmetic or pharmaceutical composition according to Claim 1, characterised in that the ratio by weight of the algae extract to the unroasted coffee extract is comprised between 1:1 and 30:1, preferably between 2:1 and 20:1 and even more preferably between 3:1 and 15:1.

3. A composition according to one or other of Claims 1 and 2, characterised in that it comprises from 0.01% to 8%,

preferably from 0.02 to 5% and even more preferably from 0.05 to 5% by weight of unroasted coffee extract.

**4.** A composition according to any one of Claims 1 to 3, characterised in that it contains from 0.1 to 15%, preferably from 0.2 to 12% and even more preferably from 0.5 to 8% by weight of algae extract.

**5.** A composition according to any one of Claims 1 to 4, characterised in that the extract of algae is obtained by hydroglycolic extraction from a mixture comprising at least 5% and preferably at least 10% of each of the chlorella, scenedesmus and spiruline algae.

**6.** A composition according to any one of Claims 1 to 5, characterised in that the green coffee extract has a caffeine concentration in excess of 40%, preferably greater than 45% and even more preferably greater than 50% in relation to the dry matter of the extract.

**7.** A composition according to any one of Claims 1 to 6, characterised in that it further contains from 0.01% to 6% by weight tocopherols, preferably constituted by tocopherol linoleate.

**8.** A composition according to any one of Claims 1 to 7, characterised in that it further contains from 0.001% to 0.1% of a complexing agent, preferably constituted by sodic EDTA.

**9.** A composition according to any one of Claims 1 to 8, characterised in that it takes the form of a lotion, an emulsion, a milk, microspheres, microgranules, a cream, a gel, a balm or an aerosol bomb, liposomes or vesicles of the ionic or non-ionic type.

**10.** Use of a hydroglycolic extract of chlorella, scenedesmus and spiruline algae in association with an unroasted coffee extract for the preparation of cosmetic or pharmaceutical compositions intended for treating the skin or capillary system and for anti-inflammatory treatments.

**11.** A method for the cosmetic treatment of the skin and capillary system, characterised in that an effective quantity of a hydroglycolic extract of chlorella, scenedesmus and spiruline algae in association with an unroasted coffee extract is applied to the skin or the capillary system.

**Patentansprüche**

**1.** Kosmetisches oder pharmazeutisches Präparat zur topischen Verwendung, **dadurch gekennzeichnet**, daß es mindestens einen Hydroglykolextrakt aus den Algen *Chlorella*, *Scenedesmus* und *Spirulina* und mindestens einen Extrakt aus grünem Kaffee in wirksamer Menge enthält.

**2.** Kosmetisches oder pharmazeutisches Präparat nach Anspruch 1, dadurch gekennzeichnet, daß das Gewichtsverhältnis zwischen dem Algenextrakt und dem Extrakt aus grünem Kaffee zwischen 1/1 und 30/1 liegt, vorzugsweise zwischen 2/1 und 20/1 und in besonders bevorzugter Weise zwischen 3/1 und 15/1.

**3.** Präparat nach einem der Ansprüche 1 und 2, dadurch gekennzeichnet, daß es 0,01 Gew. % bis 8 Gew. %, vorzugsweise 0,02 bis 5 Gew. % und in besonders bevorzugter Weise 0,05 bis 5 Gew. % an Extrakt aus grünem Kaffee enthält.

**4.** Präparat nach einem der Ansprüche 1 bis 3, dadurch gekennzeichnet daß es 0,1 Gew. % bis 15 Gew. %, vorzugsweise 0,2 bis 12 Gew. % und in besonders bevorzugter Weise 0,5 bis 8 Gew. % an Algenextrakt enthält.

**5.** Präparat nach einem der Ansprüche 1 bis 4, dadurch gekennzeichnet, daß der Algenextrakt durch Hydroglykolextraktion aus einem Gemisch erhalten wird, welches jede der Algen Chlorella, Scenedesmus und Spirulina in einer Menge von mindestens 5 %, vorzugsweise mindestens 10 %, enthält.

**6.** Präparat nach einem der Ansprüche 1 bis 5, dadurch gekennzeichnet, daß in dem Extrakt aus grünem Kaffee Kaffeesäure in einer Konzentration vorliegt, die, bezogen auf die Trockenmasse des Extrakts, höher ist als 40 %, vorzugsweise höher als 45 % und in besonders bevorzugter Weise höher ist als 50 %.

**7.** Präparat nach einem der Ansprüche 1 bis 6, dadurch gekennzeichnet, daß es außerdem 0,01 bis 6 Gew. % Tocopherole enthält, die vorzugsweise aus Tocopherol-Linoleat bestehen.

8. Präparat nach einem der Ansprüche 1 bis 7, dadurch gekennzeichnet, daß es des weiteren 0,001 % bis 0,1 % eines Komplexbildners enthält, der vorzugsweise aus Natrium-EDTA besteht.

9. Präparat nach einem der Ansprüche 1 bis 8, dadurch gekennzeichnet, daß es in Form einer Lotion, einer Emulsion, einer Milch, von Mikrokügelchen, von Mikrogranulat, einer Creme, eines Gels, eines Balsams oder eines Aerosolsprays, von Liposomen oder ionischen bzw. nicht-ionischen Bläschen vorliegt.

10. Verwendung eines Hydroglykolextrakts aus den Algen *Chlorella*, *Scenedesmus* und *Spirulina* in Verbindung mit einem Extrakt aus grünem Kaffee zur Herstellung kosmetischer oder pharmazeutischer Präparate, die zur Behandlung der Haut oder des Haarsystems und zur Behandlung zur Entzündungsbekämpfung bestimmt sind.

11. Verfahren zur kosmetischen Behandlung der Haut und des Haarsystems, dadurch gekennzeichnet, daß auf die Haut bzw. das Haarsystem eine wirksame Menge eines Hydroglykolextrakts aus den Algen *Chlorella*, *Scenedesmus* und *Spirulina* in Verbindung mit einem Extrakt aus grünem Kaffee aufgetragen wird.

FIG.1.

ARL

Protection des Lipides

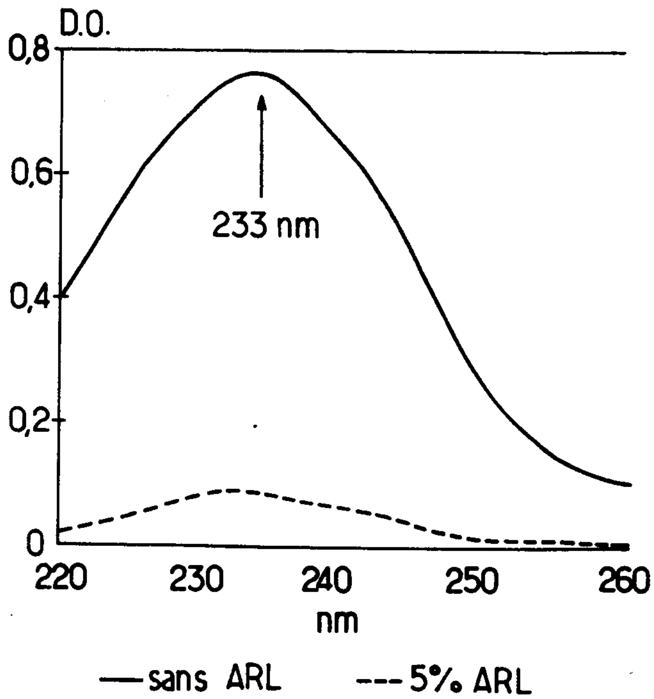


FIG.2.

ARL

Indice antiradicalaire "in vivo"